# EUROPÄISCHE PATENTSCHRIFT

(11) **EP 0 794 170 B1**
(45) Veröffentlichungstag und Bekanntmachung des Hinweises auf die Patenterteilung: **31.10.2001**
(21) Anmeldenummer: 97102725.5
(22) Anmeldetag: 20.02.1997
(51) Int. Cl.: C07C 209/36, C07C 209/84

(54) **Verfahren zur Schwersiederabtrennung bei der Diamono-toluol-Herstellung**
Process for removing high boiling compounds in the preparation of diaminotoluene
Procédé d'élimination des composés à point d'ébullition élevé dans la préparation de diaminotoluène

(30) Priorität: 05.03.1996 DE 19608443
(43) Veröffentlichungstag der Anmeldung: 10.09.1997
(73) Patentinhaber: BAYER AG, 51368 Leverkusen (DE)
(72) Erfinder: Beckhaus, Heiko, Dr., 51381 Leverkusen (DE); Witt, Harro, Dr., 25712 Kuden (DE); Zarnack, Uwe-Jens, Dr., 25541 Brunsbüttel (DE); Greger, Gerd, 47906 Kempen (DE)

(56) Entgegenhaltungen:
- DE-C- 4 230 098
- KIRK-OTHMER: " Encyclopedia of Chemical Technology, 3rd ed., vol. 2, 1978" , JOHN WILEY & SONS , NEW YORK XP002031539 * Seite 326, Zeile 1 - Zeile 19; Abbildung 3 *

## Beschreibung

Die Erfindung betrifft ein neues Verfahren zur destillativen Aufarbeitung von Amingemischen, wie sie bei der Hydrierung von technischen Dinitroaromaten anfallen, wobei bei der Isomerentrennung die höher als meta-Diaminotoluol (nachfolgend m-TDA genannt) siedenden organischen Verbindungen (Schwersieder) als Sumpfprodukt mit nur geringem Anteil m-TDA und hohem Anteil des leichtflüchtigen ortho-Diaminotoluol (nachfolgend o-TDA genannt) anfallen.

Es ist bekannt (vgl. z.B. DE-A-1 542 544, BE-B-0 631 964, BE-B-0 661 047, BE-B-0 661 946, FR-B-1 359 438 oder GB-B-0 768 111), daß man aromatische Diamine durch katalytische Hydrierung von entsprechenden aromatischen Dinitroverbindungen herstellen kann. Die Hydrierung kann unter Mitverwendung von Lösungsmitteln wie beispielsweise niedrigsiedenden Alkoholen wie Methanol, Ethanol oder Isopropanol oder gesättigten Kohlenwasserstoffen wie z.B. Tetrahydrofuran aber auch ohne solche Fremdlösungsmittel erfolgen. Die Hydrierung kann mit Hilfe von im Reaktionsgemisch dispergierten Katalysatoren durchgeführt werden, die dann durch Sedimentation oder Filtration abgetrennt und gegebenenfalls in den Prozeß zurückgeführt werden.

Aus DE-C-4 230 098 ist bekannt geworden, daß die Abtrennung von Schwersiedern unter Zusatz von den Hydriergemischen fremden Hilfsstoffen bzw. Weichmachern durchgeführt werden kann, womit sogar m-TDA-Anteile im Sumpf von bis zu 1-5 Gewichtsprozenten erzielt wurden. Das Verhältnis von Schwersiedern zu Weichmachern beträgt bestenfalls nur 1:2. Neben den Kosten für die nicht unerheblichen Mengen an Weichmachern muß ein großer Energieaufwand zur Aufheizung des Gemisches auf 290°C und zusätzlicher Entsorgungsaufwand verbunden werden.

Bislang erfolgte die Aufarbeitung des Reaktionsgemisches, indem man ein nach Abtrennung des gegebenenfalls mitverwendeten Hilfslösungsmittels und Reaktionswassers vorliegendes Gemisch aus aromatischen Diaminen mit noch vorliegenden organischen Verunreinigungen einer Isomerentrennung in einer TDA-Destillationskolonne unter Vakuum unterzieht, wobei das Hauptprodukt als Sumpfprodukt ausgetragen wird. Die leichtsiedenden Nebenverbindungen wie z.B. kernhydriertes TDA und die orthoständigen Isomeren des TDA werden über den Kopf der Kolonne abgetrennt und einer weiteren chemischen oder thermischen Verwendung zugeführt.

So ist in Kirk-Othmer, Encyclopedia Chem. Technol., 3rd ed.,vol 2, 1978, Seite 326 die destillative Aufarbeitung von roh-TDA, das durch katalytische Hydrierung von Dinitrotoluol-Isomerengemischen hergestellt wird, beschrieben. Dabei werden in der letzten, separaten Destillationsstufe die Schwersieder im m-TDA nur aufkonzentriert. Das Verfahren ist umständlich und der Verlust an m-TDA erheblich.

Es war die der Erfindung zugrundeliegende Aufgabe, ein Verfahren zur Abtrennung der schwersiedenden Nebenverbindungen von den destillierbaren m-TDA-Isomeren bei der Diaminotoluol-Herstellung zur Verfügung zu stellen, bei welchem ein thermisch verwertbarer organischer Rückstand anfällt, der nur einen geringen Anteil des Hauptproduktes m-TDA enthält.

Diese Aufgabe konnte mit dem nachstehend näher erläuterten erfindungsgemäßen Verfahren gelöst werden. Außerdem ist der zusätzlich energetische Aufwand der Schwersiederabtrennung durch die Verwendung eines nicht artfremden Schleppmittels sehr gering.

Gegenstand der Erfindung ist ein Verfahren zur Aufarbeitung von Amingemischen bei der Diaminotoluol-Herstellung aus der Hydrierung von aromatischen Dinitroverbindungen, bei dem man nach Entfernung von gegebenenfalls Lösungsmittel und Reaktionswasser aus dem TDA-Isomeren-Gemisch über eine TDA-Isomeren Destillationskolonne die leichtsiedenden TDA-Isomeren als o- und m-Isomerenfraktion destillativ abtrennt wobei man
a) die zurückbleibende, m-TDA und Schwersieder im Gemisch enthaltene Sumpfphase abtrennt und auf ca. 25-60 gew.-% Schwersiedergehalt aufkonzentriert,
b) die aufkonzentrierte Sumpfphase in einem Gewichtsverhältnis von 1:1 bis 1:5 mit einem ortho-TDA-Isomerengemisch abmischt und ein m/o-TDA-Gemisch abdestilliert und anschließend
c) das nach b) erhaltene m/o-TDA-Gemisch in die TDA-Isomerendestillationskolonne zurückführt.

Das bei dieser Aufarbeitung letztlich zurückbleibende Sumpfprodukt wird zusammen mit den thermisch zu verwertenden Organika abgemischt und nach Zwischenlagerung thermisch genutzt, wobei in vorteilhafter Weise dieses Sumpfprodukt einen m-TDA-Gehalt unter 10 Gew.-%. aufweist.

In einer bevorzugten Ausführungsform der Erfindung wird in Schritt a) die Sumpfphase in einem Verdampfer und/oder einer Abtriebskolonne mit 2 bis 10 theoretischen Böden aufkonzentriert.

Weiterhin ist es vorteilhaft, in Schritt b) das m/o-TDA-Gemisch in einer Destillationskolonne mit 2 bis 30, insbesondere 2 bis 10, theoretischen Böden abzudestillieren.

In einer anderen bevorzugten Fahrweise des erfindungsgemäßen Verfahrens weist die nach Schritt b) zurückbleibende Sumpfphase nach Abdestillieren des meta/ortho-TDA-Gemisches ein Gewichtsverhältnis von Schwersieder zu ortho-TDA von 5:1 bis 1:10 auf.

Ausgangsmaterialien für das erfindungsgemäße Verfahren sind wäßrige Aminlösungen, wie sie bei der Hydrierung von Dinitroaromaten anfallen. Besonders bevorzugt werden beim erfindungsgemäßen Verfahren Amingemische eingesetzt, wie sie bei der Hydrierung von technischen Dinitrotoluolen anfallen, und die von gegebenenfalls bei der Hydrierung mitverwendeten Hilfslösungsmittel, wie den obengenannten einfachen Alkoholen, genauso wie vom Reaktionswasser vorab destillativ befreit worden sind. Bei diesen Diaminen handelt es sich z.B. um reines 2,4-Diaminotoluol oder im allgemeinen um dessen technische Gemische mit den TDA-Isomerenzusammensetzungen von ca. 92 bis 96 Gew.-% meta, 3 bis 5 Gew.-% ortho, <1 Gew.-% para und 0,2 bis 2 Gew.-% Schwersiedern, wobei sich die Prozentsätze jeweils zu 100 Gew.-% ergänzen.

Zur Durchführung des erfindungsgemäßen Verfahrens wird eine beispielsweise mit einer Gewebepackung bestückte TDA-lsomerendestillationskolonne verwendet. Vorzugsweise weist die Kolonne 30 bis 60, insbesondere 45 bis 55, theoretische Böden auf. Die Kolonne wird bei einer Sumpftemperatur von 180 bis 220°C und unter einem Druck (am Kopf der Kolonne) von 3 000 bis 120 000 Pa betrieben, wobei diese Parameter selbstverständlich von der Natur der aufzuarbeitenden Gemische und von der angestrebten Brüdentemperatur abhängen. So kann die Kondensationswärme der Brüden in Abhängigkeit zur Energierückgewinnung durch einen Dephlegmator am Kopf der Kolonne, entsprechend dem zu gewinnenden Dampf, variieren.

Das entwässerte, rohe TDA-Gemisch kann flüssig oder gasförmig der TDA-lsomerendestillationskolonne zugeführt werden, wobei die leichtsiedenden TDA-Isomeren als reine ca. 95 bis 99 Gew.-% m/o-Isomerenfraktion abgetrennt werden. Im Falle der flüssigen Aufgabe bleibt ein Kolonnensumpf mit ca. 98 Gew.-% m-TDA und ca. 2 Gew.-% Schwersiedern zurück. Der Kolonnensumpf wird in einer nachgeschalteten Verdampferstufe auf ca. 50 Gew.-% Schwersieder aufkonzentriert, wobei das m-TDA abdestilliert und kondensiert zur weiteren Verwendung abläuft. Das Schwersiederkonzentrat wird mit in der Destillationskolonne abdestilliertem o-TDA über ein Mischorgan so abgemischt, daß in einem nachgeschalteten Verdampferschritt das gestrippte Gemische m/o-TDA abgetrennt wird und diese Brüden, vorzugsweise kondensiert, an geeigneter Stelle in die TDA-lsomerendestillationskolonne zurückgeführt werden. Im Sumpfprodukt mit ca. 50 Gew.-% Schwersiedern und 10 Gew.-% m-TDA wird das m-TDA somit vorteilhaft weitgehend durch das o-TDA, mit ca. 40 Gew.-% enthalten, ausgetauscht.

Verdampft man die TDA-Isomeren nach der Entwässerung in einer Vorkonzentrierung soweit auf; daß der Ablauf ca. 50 Gew.-% Schwersieder enthält, so werden die TDA-Isomerenbrüden der Destillationskolonne zugeführt und entsprechend in die ortho- und meta-TDA-Isomeren aufgetrennt. Der Sumpf der Kolonne enthält dann das von den Schwersiedern befreite m-TDA, was zur weiteren Verarbeitung geführt wird. Das bei der Vorkonzentrierstufe anfallende Schwersiederkonzentrat wird, wie oben beschrieben, mit abdestilliertem o-TDA über ein Mischorgan so abgemischt, daß in einem nachgeschalteten Verdampferschritt das Gemisch m/o-TDA abgetrennt wird und diese Brüden vorzugsweise kondensiert an geeigneter Stelle in die Destillationskolonne zurückgeführt werden.

Das folgende Beispiel dient zur weiteren Erläuterung des erfindungsgemäßen Verfahrens.

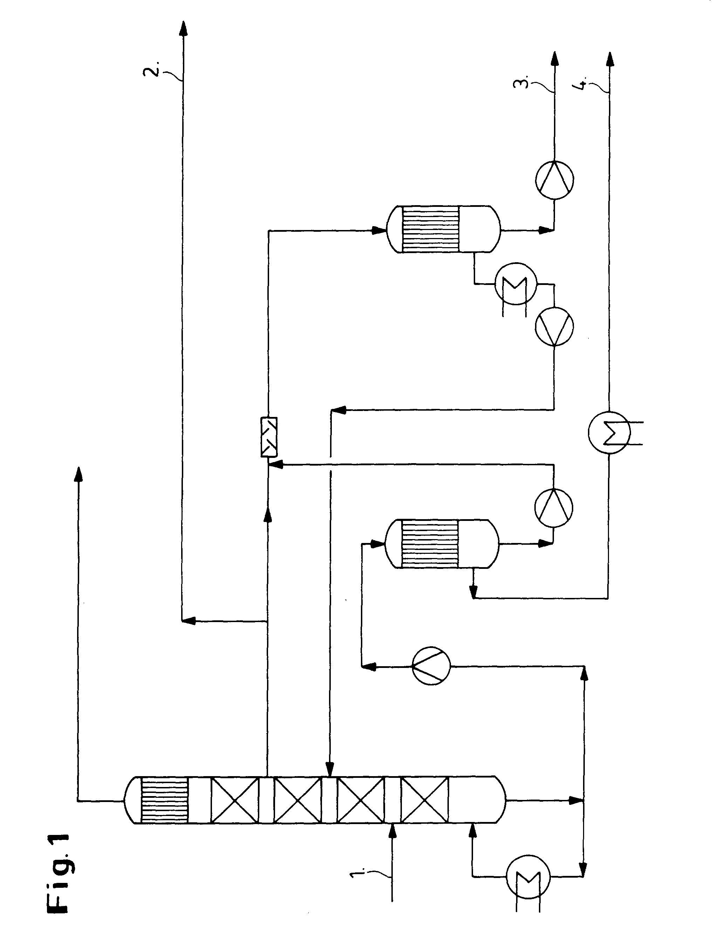

### Beispiel 1 (Schema der Aufarbeitung gemäß Fig. 1)

In eine Packungskolonne DN 50 mit 50 theoretischen Trennstufen gibt man 2,3 kg/h eines lösungsmittelfreien, entwässerten Reaktionsgemisches aus der DNT-Hydrierung auf den 8. Boden auf. Es handelt sich bei dem Gemisch um eine Zusammensetzung von im wesentlichen TDA-Isomeren mit 76,5 Gew.-% 2,4-Diaminotoluol, 18,5 Gew.-% 2,6-Diaminotoluol und 4 Gew.-% kernhydriertem TDA und 0,9 Gew.-% Schwersiedern (Strom 1). Die Kolonne wird unter einem Druck am Kopf von 8 000 Pa und einer Sumpftemperatur 203°C betrieben. Das o-TDA wird bei einer Kopftemperatur von 160°C total kondensiert und 0,3 kg/h 99 gew.-%ig entsprechend temperaturgeregelt, flüssig auf 45. Boden entnommen. Die 20 g/h durch den Dephlegmator durchschlagenden Brüden, die ca. 30 Gew.-% kernhydriertes TDA in o-TDA enthalten, werden kondensiert und bei ca. 100°C in die Vorlage der thermischen Verwertung abgeleitet.

Der Sumpf, bestehend aus 99 Gew.-% 2,4/2,6-Diaminotoluolgemisch und 1 Gew.-% Schwersiedern, wird in einem nachgeschalteten Fallfilmverdampfer soweit unter gleichem Unterdruck vom m-TDA befreit, daß die Schwersieder bis auf ca. 50 Gew.-% aufkonzentriert werden. Die Brüden, 2,15 kg/h m-TDA, werden kondensiert und zur Weiterverarbeitung oder zum Verkauf ausgelagert (Strom 4). Das Konzentrat (70 g/h) wird mit flüssig entnommenem o-TDA 250 g/h im Gewichtsverhältnis 1:3,5 abgemischt und die TDA-Isomeren gemeinsam bei einem Druck von 8 000 Pa verdampft. Die Brüden (250 g/h) werden kondensiert und am 25. Boden flüssig in die Isomerenkolonne zurückgeführt. Das Sumpfprodukt, 70 g/h bestehend aus 50 Gew.-% Schwersiedern, 40 Gew.-% o-TDA und 10 Gew.-% m-TDA (Strom 3) wird in die Vorlage zur thermischen Verwertung gegeben, wobei sich ein organisches Gemisch mit dem Hauptbestandteil o-TDA (Strom 2) zur Verbrennung mit einem Heizwert von ca. 33.000 kcal/kg ergibt.

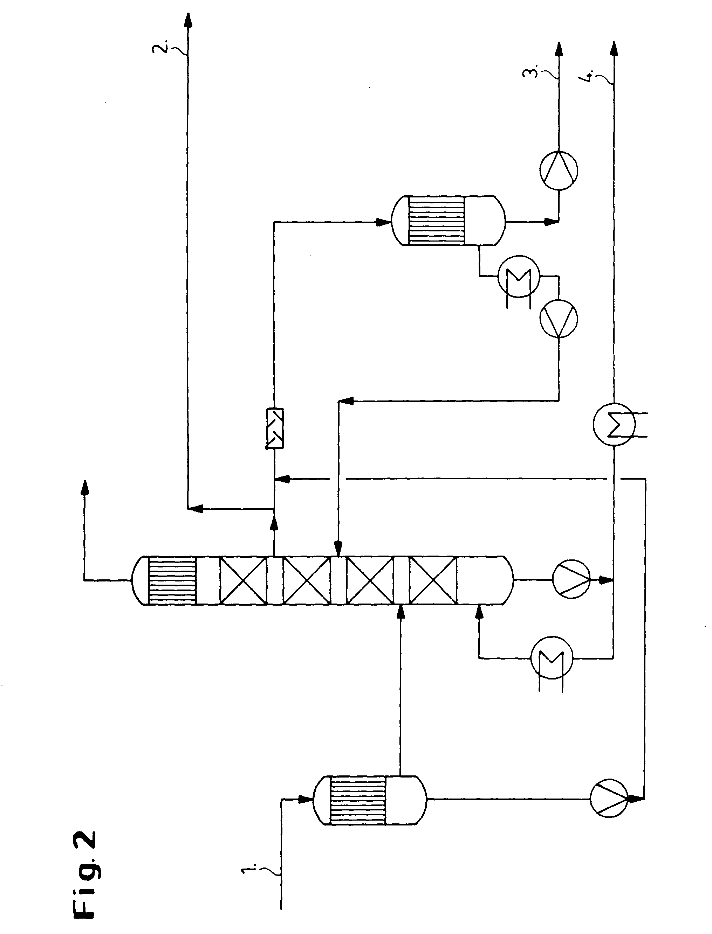

### Beispiel 2 (Schema der Aufarbeitung gemäß Fig. 2)

In eine Packungskolonne DN 50 mit 50 theoretischen Trennstufen gibt man 2,2 kg/h eines Brüdenstromes aus einem Fallfilmverdampfer, der der Isomerenkolonne vorgeschaltet ist, bestehend aus 77,9 Gew.-% 2,4-Diaminotoluol, 17,6 Gew.-% 2,6-Diaminotoluol, 4,2 Gew.-% restliche Diaminotoluole und 0,1 Gew.-% kernhydriertes TDA (Strom 1). Die TDA-Isomeren werden wie im Beispiel 1 destillativ bei einem Kolonnenkopfdruck von 4 000 Pa und Sumpftemperatur 192°C aufgetrennt, wobei das Sumpfprodukt direkt von Schwersiedern befreit auf ca. 120°C gekühlt zur Weiterverarbeitung oder zum Verkauf gelagert wird (Strom 4).

Das vom vorgeschalteten Fallfilmverdampfer ablaufende Schwersiederkonzentrat (70 g/h) wird mit flüssig entnommenem ortho-TDA (250 g/h) im Gewichtsverhältnis 1:3,5 abgemischt und die TDA-Isomeren gemeinsam bei einem Druck von 4 000 Pa verdampft. Die Brüden (250 g/h) werden kondensiert und am 25. Boden flüssig in die Isomerenkolonne zurückgeführt. Das Sumpfprodukt, 70 g/h bestehend aus 50 Gew.-% Schwersiedern, 40 Gew.-% o-TDA und 10 Gew.-% m-TDA (Strom 3) wird in die Vorlage zur thermischen Verwertung gegeben, wobei sich ein organisches Gemisch mit dem Hauptbestandteil o-TDA (Strom 2) zur Verbrennung mit einem Heizwert von ca. 33.000 kcal/kg ergibt.

## Patentansprüche

1. Verfahren zur Aufarbeitung von Amingemischen bei der Diaminotoluol-Herstellung aus der Hydrierung von aromatischen Dinitroverbindungen, bei dem man nach Entfernung von Reaktionswasser und gegebenenfalls Lösungsmittel aus dem Diaminotoluol-Isomeren-Gemisch (TDA-Gemisch) über eine TDA-Isomerendestillationskolonne die leichtsiedenden TDA-Isomeren destillativ abtrennt, **dadurch gekennzeichnet**,
a) daß man die zurückbleibende, m-TDA und Schwersieder im Gemisch enthaltene Sumpfphase abtrennt und auf ca. 25-60 gew.-% Schwersiedesgehalt aufkonzentriert,
b) die aufkonzentrierte Sumpfphase in einem Gewichtsverhältnis von 1:1 bis 1:5 mit einem ortho-TDA-Isomerengemisch abmischt und ein m/o-TDA-Gemisch abdestilliert und anschließend
c) das nach b) erhaltene m/o-TDA-Gemisch in die TDA-lsomerendestillationskolonne zurückführt.

2. Verfahren gemäß Anspruch 1, **dadurch gekennzeichnet, daß** man in Schritt a) die Sumpfphase in einem Verdampfer und/oder einer Abtriebskolonne mit 2 bis 10 theoretischen Böden aufkonzentriert.

3. Verfahren gemäß einem der Ansprüche 1 bis 2, **dadurch gekennzeichnet, daß** man in Schritt b) das m/o-TDA-Gemisch in einer Destillationskolonne mit 2 bis 30, insbesondere 2 bis 10, theoretischen Böden abdestilliert.

4. Verfahren gemäß einem der Ansprüche 1 bis 3, **dadurch gekennzeichnet, daß** in die nach Schritt b) zurückbleibende Sumpfphase nach Abdestillieren des meta/ortho-TDA-Gemisches ein Gewichtsverhältnis von Schwersieder zu ortho-TDA von 5:1 bis 1:10 aufweist.

5. Verfahren gemäß einem der Ansprüche 1 bis 4, **dadurch gekennzeichnet, daß** man die einzelnen Destillationsstufen bei einem Kolonnenkopfdruck von 3 000 bis 120 000 Pa betreibt.

## Claims

1. Process for working up amine mixtures during the production of diaminotoluene from the hydrogenation of aromatic dinitro compounds, in which, after removal of the water of reaction and optionally solvent from the mixture of diaminotoluene isomers (TDA mixture), the low-boiling TDA isomers are separated by distillation using a TDA-isomer distillation column, **characterised in that**
a) the remaining bottom phase containing a mixture of m-TDA and high-boiling components is separated and concentrated to a high-boiling component content of approximately 25 to 60 wt.%,
b) the concentrated bottom phase is mixed with an ortho-TDA isomer mixture in a weight ratio of 1:1 to 1:5 and an m/o-TDA mixture is distilled off and then
c) the m/o-TDA mixture obtained in b) is returned to the TDA-isomer distillation column.

2. Process according to claim 1, **characterised in that** in stage a) the bottom phase is concentrated in an evaporator and/or stripping column having 2 to 10 theoretical plates.

3. Process according to one of claims 1 and 2, **characterised in that** in stage b) the m/o-TDA mixture is distilled off in a distillation column having 2 to 30, in particular 2 to 10, theoretical plates.

4. Process according to one of claims 1 to 3, **characterised in that** the bottom phase remaining after stage b) after the removal of the meta/ortho-TDA mixture by distillation has a weight ratio of high-boiling components to ortho-TDA of 5:1 to 1:10.

5. Process according to one of claims 1 to 4, **characterised in that** the individual distillation stages are operated at a column overhead pressure of 3,000 to 120,000 Pa.

## Revendications

1. Procédé pour traiter les mélanges d'amines obtenus à la préparation du diaminotoluène par hydrogénation de dérivés aromatiques dinitrés, dans lequel, après élimination de l'eau de réaction et le cas échéant du solvant à partir du mélange des isomères du diaminotoluène (mélange des TDA), on sépare par distillation, sur une colonne de distillation des isomères du TDA, les isomères volatils du TDA, ce procédé se caractérisant en ce que
a) la phase résiduelle de pied de colonne contenant le m-TDA et les produits lourds en mélange, est séparée et concentrée à un niveau d'environ 25 à 60 % en poids de produits lourds,
b) la phase de pied de colonne concentrée est mélangée dans des proportions relatives en poids de 1:1 à 1:5 avec un mélange des isomères ortho du TDA ; à partir de ce mélange on distille un mélange m/o-TDA, puis
c) on recycle le mélange m/o-TDA obtenu en b) ci-dessus à la colonne de distillation des isomères du TDA.

2. Procédé selon la revendication 1, **caractérisé en ce que**, au stade opératoire a), on concentre la phase de pied de colonne dans un vaporiseur et/ou une colonne d'entraînement à 2 à 10 plateaux théoriques.

3. Procédé selon une des revendications 1 à 2, **caractérisé en ce que**, au stade opératoire b), on distille le mélange m/o-TDA dans une colonne à distiller à 2 à 30, plus spécialement 2 à 10 plateaux théoriques.

4. Procédé selon une des revendications 1 à 3, **caractérisé en ce que** la phase pied de colonne résiduelle du stade opératoire b), après distillation du mélange méta/ortho-TDA, contient les produits lourds et l'ortho-TDA dans des proportions relatives en poids de 5:1 à 1:10.

5. Procédé selon une des revendications 1 à 4, **caractérisé en ce que** les divers stades de distillation sont réalisés à une pression en tète de colonne de 3 000 à 120 000 Pa.
